# EUROPEAN PATENT SPECIFICATION

(11) **EP 4 487 830 B1**
(45) Date of publication and mention of the grant of the patent: **05.11.2025**
(21) Application number: 23766812.4
(22) Date of filing: 07.03.2023
(51) Int. Cl.: A61J 1/14, A61J 1/10, A61M 39/20, A61M 39/22, A61J 1/12

(54) **SEALING MEMBER AND BLOOD BAG SET**
DICHTUNGSELEMENT UND BLUTBEUTELSATZ
ÉLÉMENT D'ÉTANCHÉITÉ ET ENSEMBLE DE POCHES DE SANG

(30) Priority: 07.03.2022 JP 2022034092
(43) Date of publication of application: 08.01.2025
(73) Proprietor: Terumo Kabushiki Kaisha, Tokyo 151-0072 (JP)
(72) Inventor: KATAOKA, Ryoji, Fujinomiya-shi, Shizuoka 418-0004 (JP)
(74) Representative: TBK
(86) International application number: PCT/JP2023/008443
(87) International publication number: WO 2023/171636

(56) References cited:
- JP-A- H08 275 986
- JP-A- H08 275 987
- JP-U- H0 427 936
- JP-U- H0 427 936
- US-A- 6 132 413

## Description

### Technical Field

The present invention relates to a sealing member for sealing a flow path of a medical tube so as to allow the flow path of the medical tube to be in communication and a blood bag set.

### Background Art

A sealing member is used to seal a medical tube to allow the medical tube to be in communication. The sealing member is a cap-shaped member fitted in the lumen of the medical tube, and seals the medical tube in the initial state. The sealing member includes a breakable portion. In response to bending the sealing member, the breakable portion is broken and the sealing member allows the medical tube to be in communication.

Such a sealing member is used for, for example, a blood bag set (JP 8-275986 A). An exemplary sealing member is known from JP H04 27936 U5.

### Summary of Invention

Insufficient bending of the sealing member at the communicating operation causes a poor communication in which part of the breakable portion remains connected. Even in such a poor communication, the sealing member can allow liquid such as blood to flow and pass. However, in the case of a traditional sealing member, in passing of blood through a narrow breakable portion due to a poor communication, red blood cells may be damaged to cause hemolysis.

An object of the present invention defined in claim 1 is to solve the above drawbacks.

According to one aspect of the disclosure below, provided is a sealing member including: a base having an outer circumferential face for contact with an inner face of a medical tube, the base having a base flow path for allowing fluid to pass inside of the base, the base flow path extending in a direction of an axis of the sealing member, the base flow path being open at a proximal end of the base; a neck located at a distal end of the base such that the base has an outer diameter that reduces; a plug connected to the base through the neck, the plug extending from the base toward a side of location of a distal end of the sealing member in the direction of the axis of the sealing member, the plug occluding the base flow path; and a breakable portion including a thinnest portion of a boundary between a proximal end of the plug and the neck, in which, in viewing from the side of location of the distal end of the sealing member in the direction of the axis of the sealing member, the breakable portion is non-circular in shape, the breakable portion including a plurality of tops and a plurality of connections between the plurality of tops, and a distance between the base and the axis is larger than a radius of a circle inscribed in the plurality of connections, and the base is located outside the plurality of connections in a radial direction of the sealing member.

According to another aspect of the disclosure below, provided is a blood bag set including: the sealing member of the aspect described above; a blood bag that houses whole blood or a blood component; and a medical tube in communication with the blood bag, in which the sealing member occludes the medical tube in contact with an inner face of the medical tube so as to allow the medical tube to be in communication.

According to the sealing member and the blood bag set of the aspects described above, the breakable portion is broken smoothly, thereby reducing occurrence of a poor communication. Further, according to the sealing member and the blood bag set, even in occurrence of a poor communication in the sealing member, hemolysis can be prevented because the breakable portion is large in flow-path cross-sectional area.

### Brief Description of Drawings

Fig. 1 is a side view of a sealing member according to an embodiment.
Fig. 2 is a six-sided view of the sealing member of Fig. 1.
Fig. 3 is a longitudinal sectional view of the sealing member of Fig. 1.
Fig. 4A is a perspective view of the sealing member of Fig. 1. Fig. 4B is a view of a broken portion after separation of a plug of the sealing member of Fig. 1 in viewing from the side of location of the distal end of the sealing member in the axial direction of the sealing member.
Fig. 5 is an illustration of the distribution of stress generated in the breakable portion resulting from bending of the plug of the sealing member of Fig. 1.
Fig. 6 is an explanatory view of the sealing member of Fig. 1 in occurrence of a poor communication.
Fig. 7 is an explanatory view of a sealing member in occurrence of a poor communication according to a comparative example.
Fig. 8 is a comparison table of the sealing member according to the comparative example and the sealing member of Fig. 1 in the cross-sectional shape and opening area of the breakable portion.
Fig. 9 is a schematic view of a blood bag set including the sealing member of Fig. 1.
Fig. 10A is a view of the shape of a breakable portion according to Modification 1 of the embodiment. Fig. 10B is a view of the shape of a breakable portion according to Modification 2 of the embodiment.
Fig. 11 is a sectional view of the shape of a breakable portion according to Modification 3 of the embodiment.

### Description of Embodiments

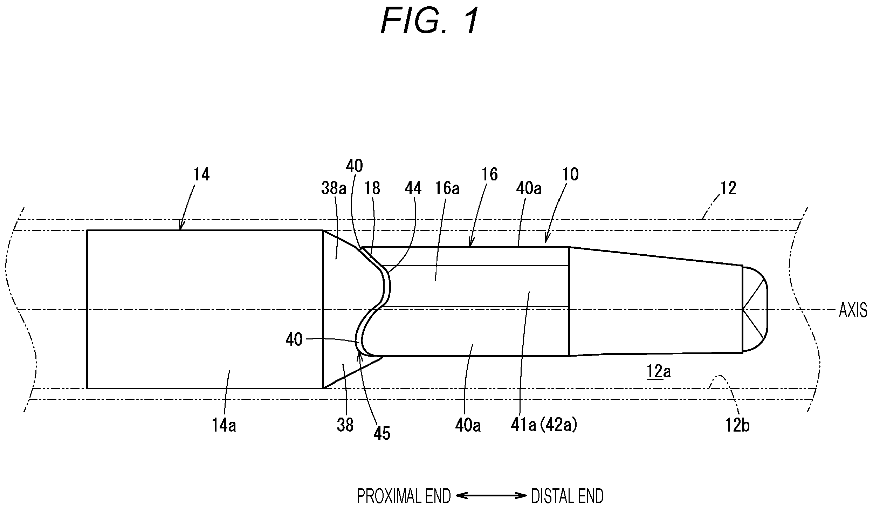

As illustrated in Fig. 1, a sealing member 10 according to the present embodiment is used for sealing a flow path 12a of a medical tube 12. The sealing member 10 is liquid-tightly secured to the flow path 12a of the medical tube 12 in the initial state. The sealing member 10 includes a base 14, a plug 16, and a breakable portion 18. In the initial state, the base 14 and the plug 16 are in connection through the breakable portion 18, and the plug 16 seals the flow path 12a. In response to bending the plug 16, the breakable portion 18 is broken, so that the sealing member 10 allows the flow path 12a to be in communication.

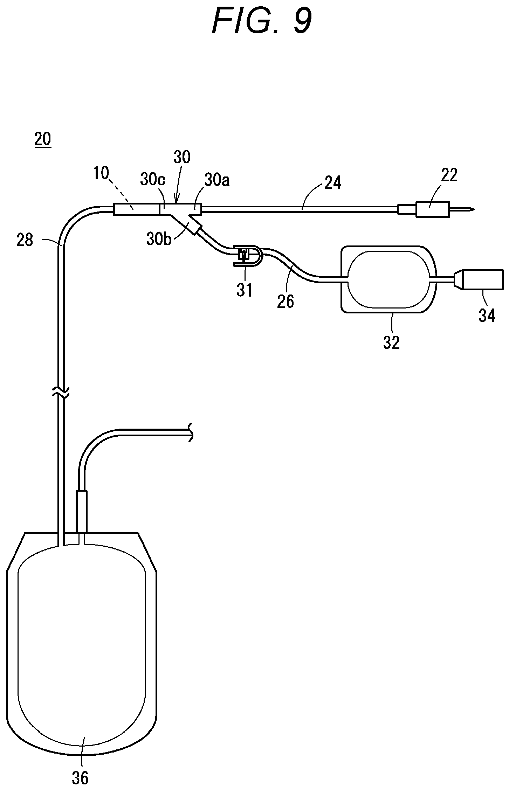

Such a sealing member 10 is used for, for example, a blood bag set 20 illustrated in Fig. 9. The blood bag set 20 includes a blood sampling needle 22, a first tube 24, a second tube 26, a third tube 28, a branch portion 30, an initial flow blood bag 32, a sampling holder 34, and a blood bag 36.

The blood sampling needle 22 is connected to one end of the first tube 24. The other end of the first tube 24 is connected to a first port 30a of the branch portion 30 branched in three directions. The second tube 26 is connected to a second port 30b (see Fig. 9) of the branch portion 30, and the third tube 28 is connected to a third port 30c (see Fig. 9) of the branch portion 30. The second tube 26 connects the branch portion 30 and the initial flow blood bag 32. The sampling holder 34 is connected downstream of the initial flow blood bag 32. The third tube 28 connects the branch portion 30 and the blood bag 36 (blood sampling bag). The sealing member 10 of Fig. 1 is attached to the third tube 28 near the third port 30c of the branch portion 30. In the initial state, the sealing member 10 occludes the third port 30c and the third tube 28.

The blood sampling needle 22 of the blood bag set 20 draws blood from a blood donor (donor). Because the third port 30c of the branch portion 30 is occluded by the sealing member 10, the drawn blood flows into the second tube 26 through the second port 30b. The blood is stored in the initial flow blood bag 32. The sampling holder 34 is used to collect a portion of the drawn blood in, for example, a sample tube.

The operation of collecting the blood of the donor into the blood bag 36 is performed by occluding the second tube 26 of the blood bag set 20 with a clamp 31 and allowing the sealing member 10 to be in communication. In such a manner, the sealing member 10 is used, for example, for allowing a flow path of the blood bag set 20 to be in communication and occluding the flow path thereof.

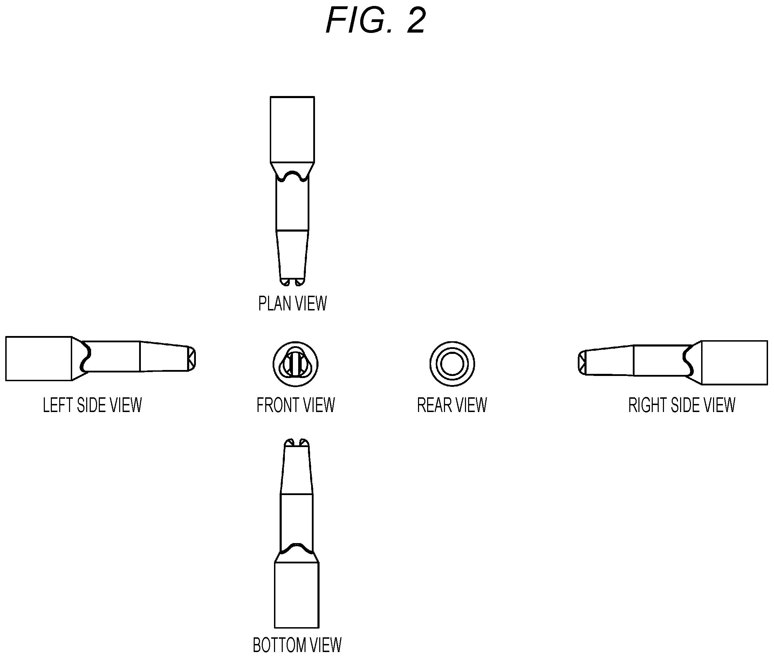

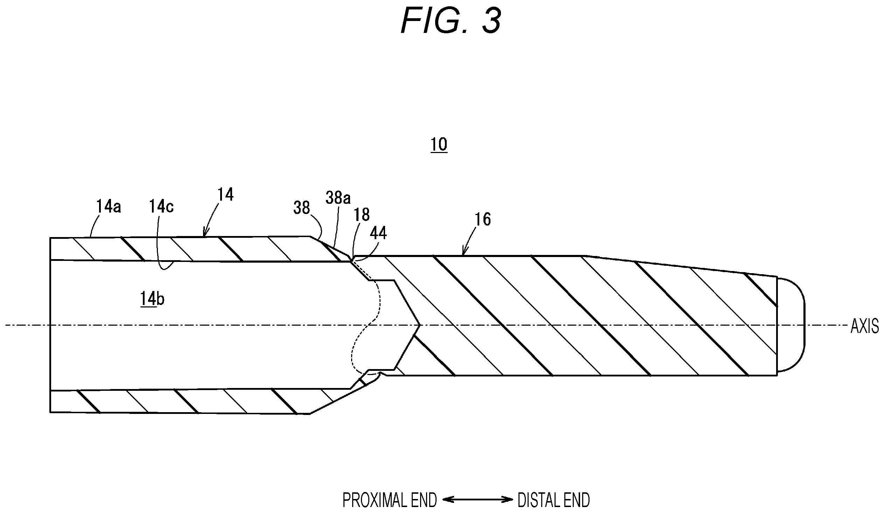

As illustrated in Figs. 1 to 4A, the sealing member 10 includes the base 14 and the plug 16. The base 14 has a cylindrical shape extending in the axial direction of the sealing member 10. An outer circumferential face 14a of the base 14 is secured to the flow path 12a in liquid-contact with an inner circumferential face 12b of the flow path 12a of the medical tube 12. The medical tube 12 is, for example, the third tube 28. As illustrated in Fig. 3, the base 14 of the sealing member 10 has a base flow path 14b inside of the base 14. The base flow path 14b is surrounded by the inner circumferential face 14c of the base 14 concentric with the outer circumferential face 14a thereof. The base flow path 14b extends in the axial direction of the sealing member 10. The proximal end of the base flow path 14b is open at the proximal end of the base 14. The distal end of the base flow path 14b extends to the inside of the plug 16.

As illustrated in Fig. 1, the base 14 has a neck 38 at its distal end. The neck 38 is an extending portion that reduces in outer diameter to its distal end such that the outer diameter is smaller than that of the base 14. The neck 38 has an outer circumferential face 38a including a conical face.

The plug 16 extends from the neck 38 toward its distal end in the axial direction of the sealing member 10. In the initial state, the plug 16 is integrally connected to the base 14 through the neck 38. As illustrated in Fig. 3, the plug 16 has a solid portion. The solid portion of the plug 16 covers the side of location of the distal end of the base flow path 14b such that the plug 16 occludes the distal end of the base flow path 14b. As illustrated in Fig. 1, the plug 16 has an outer diameter smaller than that of the base 14. Therefore, with the sealing member 10 secured to the medical tube 12, the plug 16 is separated from the inner circumferential face 12b of the medical tube 12.

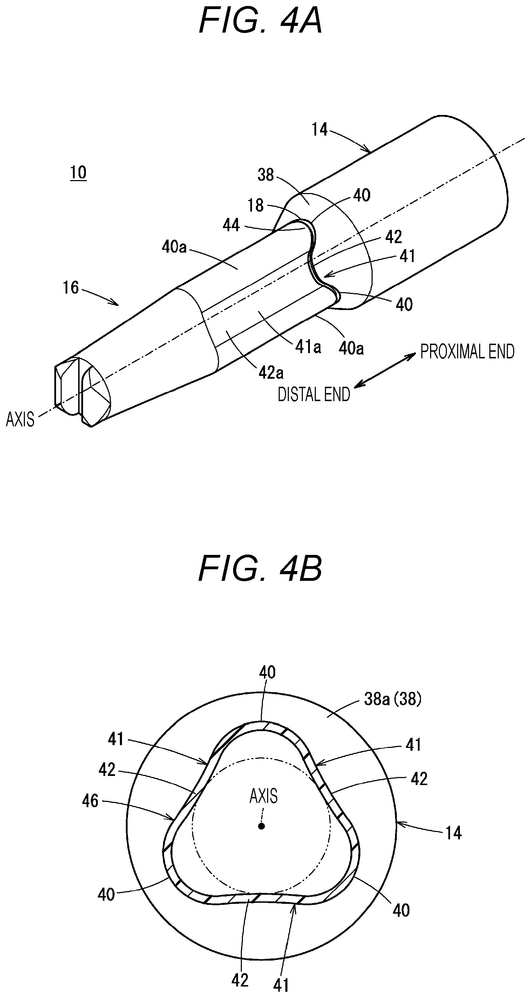

As illustrated in Fig. 4A, the plug 16 has three ridges 40a radially protruding outside from the central axis of the sealing member 10. The three ridges 40a of the plug 16 are each circumferentially disposed at an angle of 120° around the central axis. Each of the ridges 40a extends linearly in the axial direction of the sealing member 10. The breakable portion 18 includes each top 40 at the boundary between the corresponding ridge 40a and the neck 38. A relatively flat curved face 41a is located between one ridge 40a and another ridge 40a adjacent thereto. The curved face 41a is a portion having a relatively small curvature, and connects two adjacent tops 40 due to such a smooth curved face. The breakable portion 18 includes a connection 41 at the boundary between the curved face 41a and the neck 38.

As illustrated, the curved face 41a of the plug 16 may have a recessed face portion 42a curved in a recessed shape toward the central axis of the sealing member 10. The recessed face portion 42a includes a curved face having a center of curvature located outside the plug 16. The breakable portion 18 includes each recess 42 at the boundary between the corresponding recessed face portion 42a and the neck 38. The connection 41 including the recess 42 is a portion where the breakable portion 18 is relatively easily broken by the force of bending the plug 16. In the present embodiment, such an easily breakable portion appears in three directions, and thus the user can break the breakable portion 18 without concern for the orientation of the plug 16.

As illustrated in Fig. 1, the breakable portion 18 is located at the boundary between the proximal end of the plug 16 and the neck 38. The breakable portion 18 includes a groove 44 along a line where an outer circumferential face 16a of the plug 16 and an outer circumferential face 38a of the neck 38 intersect mutually. In the present embodiment, the groove 44 is the thinnest portion 45 that is the thinnest portion of the sealing member 10. The groove 44 extends linearly and extends circumferentially along the outer circumferential face 38a of the neck 38. As illustrated in Figs. 4A and 4B, in viewing from the side of location of the distal end of the sealing member 10 in the axial direction of the sealing member 10, the groove 44 has a plurality of such tops 40 and such recesses 42 (connections 41) as described above that are located between the tops 40, and extends circumferentially in a wavy manner. As illustrated in Fig. 1, the groove 44 is also serpentine in the axial direction of the sealing member 10 in side view. In such a manner, the groove 44 extends three-dimensionally without extending along one plane.

As illustrated in Fig. 3, the groove 44 of the breakable portion 18 is the thinnest of the other portions of the sealing member 10. Thus, the groove 44 is easily broken resulting from the communicating operation of bending the plug 16. In response to the communicating operation, the entire region of the groove 44 is broken and the plug 16 is separated from the base 14. The base 14 with the breakable portion 18 broken has a broken portion 46 illustrated in Fig. 4B. As illustrated, the groove 44 has the broken portion 46 circumferentially wavy in viewing from the side of location of the distal end of the sealing member 10 in the axial direction of the sealing member 10.

The sealing member 10 of the present embodiment acts as follows.

As illustrated in Fig. 1, because the plug 16 of the sealing member 10 is separated from the inner circumferential face 12b of the medical tube 12, the plug 16 can be bent. In response to application of a force of bending the plug 16 to the base 14 from the outside of the medical tube 12, the breakable portion 18 is broken. Although the usage is not limited, the recessed face portion 42a between each ridge 40a of the plug 16 is recessed, and thus the plug 16 is a portion to which the user can easily input the operation force.

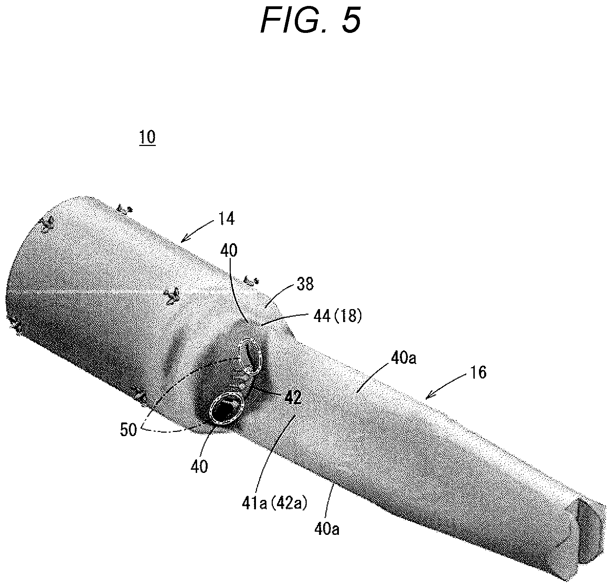

As illustrated in Fig. 5, in response to input of a force from the recess 42 toward the central axis, a stress concentration portion 50 is generated at one end and the other end in the circumferential direction of the recess 42. Such stress concentration portions 50 as described above are each a start point of breakage of the breakable portion 18 resulting from input of a relatively weak bending force. Due to expansion of the breaking part of the breakable portion 18, the breakable portion 18 of the sealing member 10 is reliably broken even with input of a weak bending force. As a result, the sealing member 10 can inhibits the occurrence frequency of a poor communication.

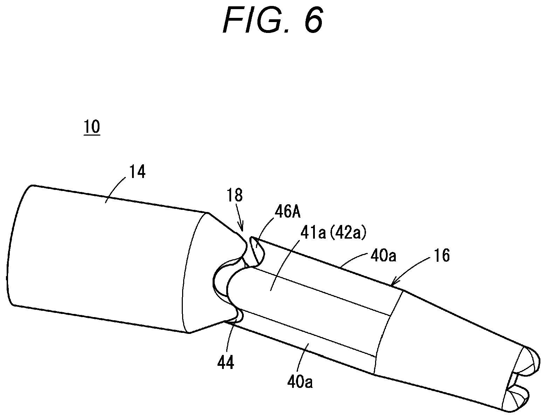

As illustrated in Fig. 6, even in a case where part of the breakable portion 18 remains without being broken, the sealing member 10 has a three-dimensionally wavy broken portion 46A. In such a sealing member 10, the gap between the neck 38 and the plug 16 along the broken portion 46A serves as a fluid flow path. The three-dimensionally wavy broken portion 46A can have a relatively large flow-path cross-sectional area. Therefore, even in occurrence of a poor communication, the sealing member 10 can prevent the red blood cells in the blood from damage, resulting in reduction of the risk of hemolysis.

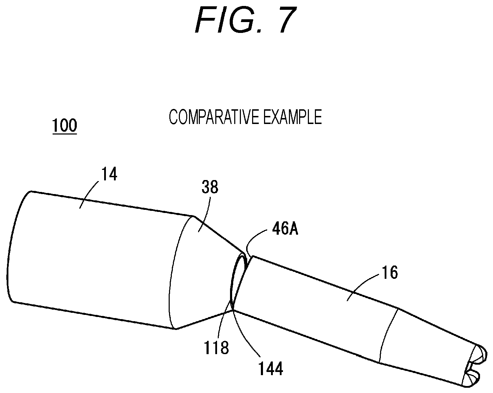

In comparison with the sealing member 10, a sealing member 100 of a comparative example in Fig. 7 includes a breakable portion 118 including a circular groove 144. In the sealing member 100 of the comparative example, a flow path formed by a broken portion 46A in occurrence of a poor communication is narrower.

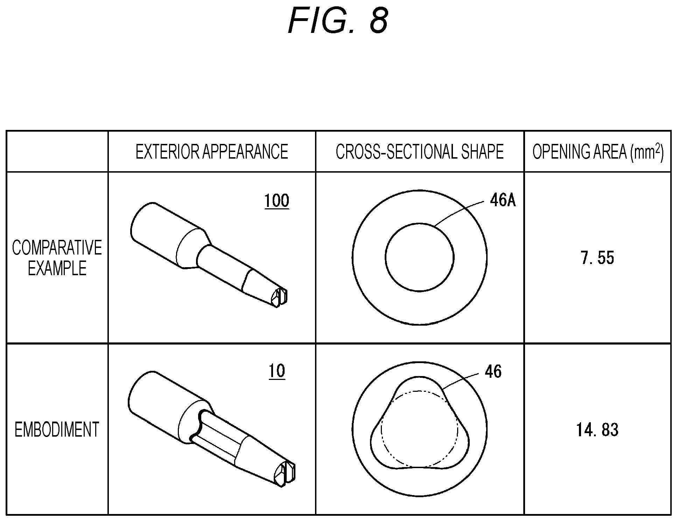

As illustrated in Fig. 8, the sealing member 100 of the comparative example has an opening area of 7.55 mm², whereas the sealing member 10 of the present embodiment can has an opening area of 14.83 mm² larger than the opening area of the sealing member 100. In the sealing member 10, the cross-sectional area of the flow path formed due to occurrence of a poor communication increases in accordance with the length of the broken portion 46, and in the sealing member 100, the cross-sectional area of the flow path formed due to occurrence of a poor communication increases in accordance with the length of the broken portion 46A. The broken portion 46 of the sealing member 10 of the present embodiment is longer than the broken portion 46A of the sealing member 100 of the comparative example. Therefore, even in occurrence of a poor communication, the sealing member 10 can has a larger flow-path cross-sectional area formed by the broken portion 46, resulting in reduction of the risk of hemolysis.

### (Modification 1 and Modification 2 of the Embodiment)

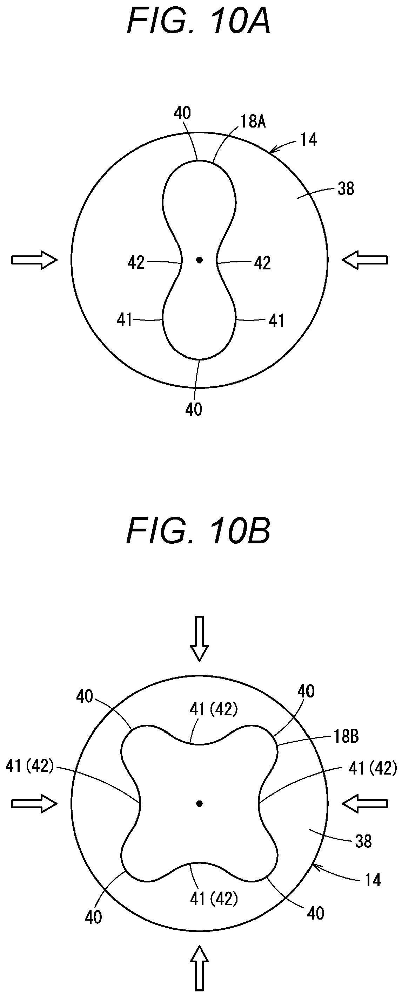

In the embodiment described above, the example in which the groove 44 of the breakable portion 18 includes the three tops 40 has been described, but the present embodiment is not limited thereto. For example, as in Modification 1 illustrated in Fig. 10A, a breakable portion 18A may include two tops 40 in viewing from the side of location of the distal end of a sealing member 10 in the axial direction of a sealing member 10. In addition, as in Modification 2 illustrated in Fig. 10B, a breakable portion 18B may include four tops 40 in viewing from the side of location of the distal end of a sealing member 10 in the axial direction of a sealing member 10.

### (Modification 3 of the Embodiment)

In the above description, described has been the thinnest portion 45 including the groove 44 (see Fig. 3) having a V-shaped cross section on the outer circumference side of the sealing member 10; however, the present embodiment is not limited thereto. In Modification 3 of the present embodiment, another configuration example of the thinnest portion 45 will be described.

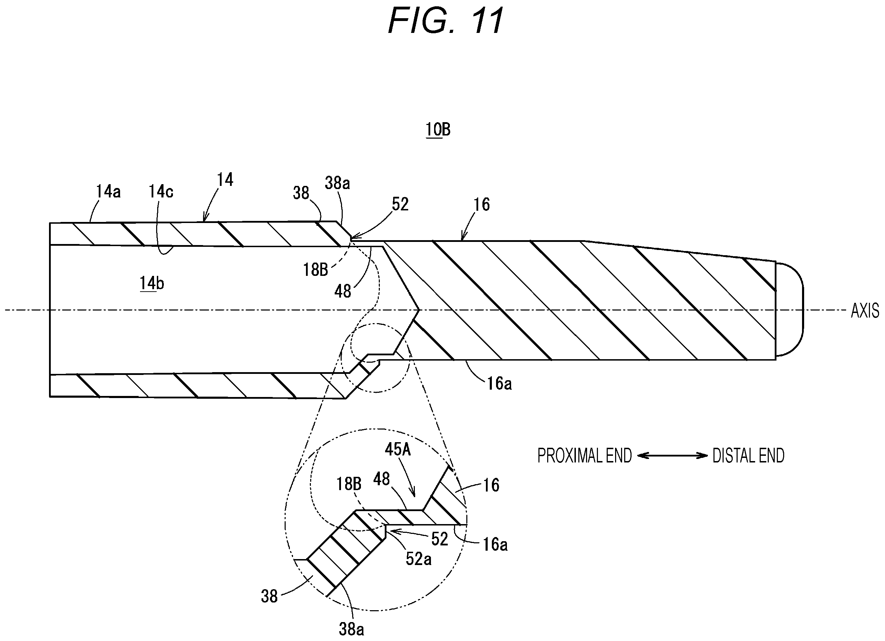

As illustrated in Fig. 11, a sealing member 10B of the present embodiment includes a thinnest portion 45A. The thinnest portion 45A includes an inner thin portion 48 that increases in diameter of a plug 16 from the inner circumference side, and a step 52 provided at the boundary between a neck 38 and the plug 16. The step 52 is located at the boundary between an outer circumferential face 38a of the neck 38 and an outer circumferential face 16a of the plug 16. The step 52 includes a step face 52a facing a direction perpendicular to the axial direction of the sealing member 10B. The step face 52a and the outer circumferential face 16a of the plug 16 intersect substantially perpendicularly with each other. On the step 52, the neck 38 reduces in thickness and the thinnest portion 45A is located. The thinnest portion 45A is located between the outer circumferential face 16a and the inner thin portion 48, and has a width in the axial direction of the sealing member 10B. The thinnest portion 45A extends over the entire region in the circumferential direction of the plug 16.

A breakable portion 18B of the present modification is provided on a step 52 of the thinnest portion 45A. The breakable portion 18B extends over the entire region in the circumferential direction. In response to application of a bending force to the plug 16, stress concentrates on the step 52 of the thinnest portion 45A. As a result, the breakable portion 18B is broken. In such a manner, in the sealing member 10B of the present modification, the breakable portion 18B of the thinnest portion 45A can be broken resulting from a bending operation on the plug 16.

The sealing member 10 and the blood bag set 20 of the present embodiment are summarized as follows.

According to the embodiment described above, provided is a sealing member 10 including: a base 14 having an outer circumferential face 14a for contact with an inner face of a medical tube 12, the base 14 having a base flow path 14b for allowing fluid to pass inside of the base 14, the base flow path extending in a direction of an axis of the sealing member 10, the base flow path being open at a proximal end of the base 14; a neck 38 located at a distal end of the base 14 such that the base 14 has an outer diameter that reduces; a plug 16 connected to the base 14 through the neck 38, the plug 16 extending from the base 14 toward a side of location of a distal end of the sealing member 10 in the direction of the axis of the sealing member 10, the plug 16 occluding the base flow path; and a breakable portion 18 including a thinnest portion 45 of a boundary between a proximal end of the plug 16 and the neck 38, in which, in viewing from the side of location of the distal end of the sealing member 10 in the direction of the axis of the sealing member 10, the breakable portion 18 is non-circular in shape, the breakable portion 18 including a plurality of tops 40 and a plurality of connections 41 between the plurality of tops 40, a distance between the base 14 and the axis is larger than a radius of a circle inscribed in the plurality of connections 41, and the base 14 is located outside the plurality of connections 41 in a radial direction of the sealing member 10.

In response to bending of the plug, the sealing member described above has a stress concentration portion 50 on the breakable portion. Thus, the breakable portion can be broken reliably even with a weak force. As a result, the frequency of occurrence of a poor communication of the breakable portion is inhibited. Further, the breakable portion has a large opening area of the gap formed by the breakable portion, even in occurrence of a poor communication with the breakable portion not partially broken. Therefore, the sealing member can reduce the risk of hemolysis.

In the sealing member described above, the breakable portion may include the thinnest portion extending in a circumferential direction over entire of the boundary between the proximal end of the plug and the neck along the boundary. The breakable portion can be broken with a force from any direction in the circumferential direction, and thus the sealing member is excellent in operability.

In viewing from the side of location of the distal end of the sealing member in the direction of the axis of the sealing member, each of the plurality of connections of the breakable portion may include a recess 42 recessed toward a center. A portion where the plug is easily bent is located in a relatively flat portion between two adjacent tops of the plurality of tops. Therefore, the breakable portion can be broken more reliably.

In viewing from the side of location of the distal end of the sealing member in the direction of the axis of the sealing member, the breakable portion may be wavy in shape in the circumferential direction due to the plurality of tops and the recess. Due to the wavy shape of the breakable portion, the opening area increases in occurrence of a poor communication.

In viewing from the side of location of the distal end of the sealing member in the direction of the axis of the sealing member, the plurality of tops of the breakable portion may be three in number. A portion where the plug is easily bent is located in a relatively flat portion between two adjacent tops of the plurality of tops. In this case, because the direction in which the plug is easily bent appears in three directions, the user can perform a bending operation without being conscious of the orientation of the plug. Further, because the relatively flat portion is large in width, the breakable portion can be easily broken even with a weak pressurizing force.

In the sealing member described above, the neck may have a conical face inclined such that the diameter of the neck gradually reduces toward the side of location of the distal end of the sealing member in the direction of the axis of the sealing member, and the breakable portion may be located on the conical face. Such a sealing member can include a breakable portion that is three-dimensionally wavy in shape at the boundary between the neck and the plug, and thus the opening area of the flow path can increase even in occurrence of a poor communication. Therefore, the sealing member can reduce the risk of hemolysis in occurrence of the poor communication.

According to the embodiment described above, provided is a blood bag set 20 including: the sealing member described above; a blood bag 36 that houses whole blood or a blood component; and a medical tube in communication with the blood bag 36, in which the sealing member occludes the medical tube in contact with an inner face of the medical tube so as to allow the medical tube to be in communication.

The blood bag set can reduce the risk of hemolysis with the sealing member.

Note that the present invention is not limited to the embodiments described above, and thus various configurations may be adopted without departing from the scope of the invention defined by the appended claims.

## Claims

1. A sealing member comprising:
a base having an outer circumferential face for contact with an inner face of a medical tube, the base having a base flow path for allowing fluid to pass inside of the base, the base flow path extending in a direction of an axis of the sealing member, the base flow path being open at a proximal end of the base;
a neck located at a distal end of the base such that the base has an outer diameter that reduces;
a plug connected to the base through the neck, the plug extending from the base toward a side of location of a distal end of the sealing member in the direction of the axis of the sealing member, the plug occluding the base flow path; and
a breakable portion including a thinnest portion of a boundary between a proximal end of the plug and the neck,
**characterized in that**
in viewing from the side of location of the distal end of the sealing member in the direction of the axis of the sealing member, the breakable portion is non-circular in shape, the breakable portion including a plurality of tops and a plurality of connections between the plurality of tops, and
a distance between the base and the axis is larger than a radius of a circle inscribed in the plurality of connections, and the base is located outside the plurality of connections in a radial direction of the sealing member.

2. The sealing member according to claim 1, wherein the breakable portion includes the thinnest portion extending in a circumferential direction over an entire of the boundary between the proximal end of the plug and the neck along the boundary.

3. The sealing member according to claim 2, wherein, in viewing from the side of location of the distal end of the sealing member in the direction of the axis of the sealing member, each of the plurality of connections of the breakable portion includes a recess recessed toward a center.

4. The sealing member according to claim 3, wherein, in viewing from the side of location of the distal end of the sealing member in the direction of the axis of the sealing member, the breakable portion is wavy in shape in the circumferential direction due to the plurality of tops and the recess.

5. The sealing member according to any one of claims 2 to 4, wherein, in viewing from the side of location of the distal end of the sealing member in the direction of the axis of the sealing member, the plurality of tops of the breakable portion is three in number.

6. The sealing member according to any one of claims 1 to 5, wherein the neck has a conical face inclined such that a diameter of the neck gradually reduces toward the side of location of the distal end of the sealing member in the direction of the axis of the sealing member, and the breakable portion is located on the conical face.

7. A blood bag set comprising:
the sealing member according to any one of claims 1 to 6;
a blood bag that houses whole blood or a blood component; and
a medical tube in communication with the blood bag, wherein
the sealing member occludes the medical tube in contact with an inner face of the medical tube so as to allow the medical tube to be in communication.

## Patentansprüche

1. Dichtungselement umfassend:
eine Basis, die eine äußere Umfangsfläche für den Kontakt mit einer inneren Fläche eines Medizinschlauches hat, wobei die Basis einen Basis-Strömungsweg hat, um einem Fluid zu ermöglichen in das Innere der Basis zu passieren, wobei sich der Basis-Strömungsweg in einer Achsrichtung des Dichtungselements erstreckt, wobei der Basis-Strömungsweg an einem proximalen Ende der Basis offen ist;
einen Hals, der sich an einem distalen Ende der Basis befindet, sodass die Basis einen sich reduzierenden Außendurchmesser hat;
einen Stopfen, der durch den Hals mit der Basis verbunden ist, wobei sich der Stopfen von der Basis zu einer Seite der Stelle eines distalen Endes des Dichtungselements in die Achsrichtung des Dichtungselementes erstreckt, wobei der Stopfen den Basis-Strömungsweg verschließt; und
einen brechbaren Abschnitt, der einen dünnsten Abschnitt von einer Grenze zwischen einem proximalen Ende des Stopfens und dem Hals aufweist, **dadurch gekennzeichnet, dass**
aus der Blickrichtung der Seite der Stelle des distalen Endes des Dichtungselements, in der Achsrichtung des Dichtungselementes, der brechbare Abschnitt eine nicht kreisartige Form hat, wobei der brechbare Abschnitt eine Vielzahl von oberen Bereichen und eine Vielzahl von Verbindungen zwischen der Vielzahl an oberen Bereichen umfasst, und
ein Abstand zwischen der Basis und der Achse größer als der Radius eines in die Vielzahl von Verbindungen eingeschriebenen Kreises ist, und sich die Basis in einer Radialrichtung des Dichtungselementes außerhalb der Vielzahl an Verbindungen befindet.

2. Dichtungselement nach Anspruch 1, wobei der brechbare Abschnitt den dünnsten Abschnitt aufweist, der sich in einer Umfangsrichtung über eine Gesamtheit der Grenze zwischen dem proximalen Ende des Stopfens und dem Hals entlang der Grenze erstreckt.

3. Dichtungselement nach Anspruch 2, wobei, aus der Blickrichtung der Seite der Stelle des distalen Endes des Dichtungselementes in die Achsrichtung des Dichtungselementes, jede der Vielzahl an Verbindungen von brechbaren Abschnitten eine Vertiefung aufweist, die in Richtung eines Zentrums vertieft ist.

4. Dichtungselement nach Anspruch 3, wobei, aus der Blickrichtung der Seite der Stelle des distalen Endes des Dichtungselementes in die Achsrichtung des Dichtungselementes, der brechbare Abschnitt aufgrund von der Vielzahl von oberen Bereichen und der Vertiefung in der Umfangsrichtung eine gewellte Form hat.

5. Dichtungselement nach einem der Ansprüche 2 bis 4, wobei, aus der Blickrichtung der Seite der Stelle des distalen Endes des Dichtungselementes in die Achsrichtung des Dichtungselementes, die Vielzahl an oberen Bereichen der brechbaren Abschnitte eine Anzahl von drei ist.

6. Dichtungselement nach einem der Ansprüche 1 bis 5, wobei der Hals eine konische Fläche besitzt, die so geneigt ist, dass sich ein Durchmesser des Halses zu der Seite der Stelle des distalen Endes des Dichtungselementes in der Achsrichtung des Dichtungselementes allmählich reduziert, und der brechbare Abschnitt sich an der konischen Fläche befindet.

7. Blutbeutelsystem umfassend:
das Dichtungselement nach einem der Ansprüche 1 bis 6;
einen Blutbeutel, der Vollblut oder eine Blutkomponente aufnimmt, wobei
das Dichtungselement den Medizinschlauch in Kontakt mit einer inneren Fläche des Medizinschlauches verschließt, um dem Medizinschlauch zu ermöglichen, in Verbindung zu stehen.

## Revendications

1. Élément de scellage comprenant :
une base ayant une face circonférentielle externe destinée à être en contact avec une face interne d'un tube médical, la base ayant un trajet d'écoulement de base permettant à un fluide de passer à l'intérieur de la base, le trajet d'écoulement de base s'étendant dans la direction d'un axe de l'élément de scellage, le trajet d'écoulement de base étant ouvert au niveau d'une extrémité proximale de la base ;
un col situé à une extrémité distale de la base de telle sorte que la base présente un diamètre externe se réduisant ;
un bouchon relié à la base par le biais du col, le bouchon s'étendant de la base vers un côté de l'emplacement d'une extrémité distale de l'élément de scellage dans la direction de l'axe de l'élément de scellage, le bouchon obturant le trajet d'écoulement de la base ; et
une partie cassable comportant une partie la plus mince d'une limite entre une extrémité proximale du bouchon et le col, **caractérisé en ce que**
lorsque l'on regarde depuis le côté de l'emplacement de l'extrémité distale de l'élément de scellage dans la direction de l'axe de l'élément de scellage, la partie cassable est de forme non circulaire, la partie cassable comportant une pluralité de sommets et une pluralité de liaisons entre les sommets de la pluralité de sommets, et
une distance entre la base et l'axe est supérieure à un rayon d'un cercle inscrit dans la pluralité de raccords, et la base est située à l'extérieur de la pluralité de raccords dans une direction radiale de l'élément de scellage.

2. Élément de scellage selon la revendication 1, dans lequel la partie cassable comporte la partie la plus mince s'étendant dans une direction circonférentielle sur toute la limite entre l'extrémité proximale du bouchon et le col le long de la limite.

3. Élément de scellage selon la revendication 2, dans lequel, lorsque l'on regarde depuis le côté de l'emplacement de l'extrémité distale de l'élément de scellage dans la direction de l'axe de l'élément de scellage, chaque liaison de la pluralité de liaisons de la partie cassable comporte un évidement en retrait vers un centre.

4. Élément de scellage selon la revendication 3, dans lequel, lorsque l'on regarde depuis le côté de l'emplacement de l'extrémité distale de l'élément de scellage dans la direction de l'axe de l'élément de scellage, la partie cassable a une forme ondulée dans la direction circonférentielle en raison de la pluralité de sommets et de l'évidement.

5. Élément de scellage selon l'une quelconque des revendications 2 à 4, dans lequel, lorsque l'on regarde depuis le côté de l'emplacement de l'extrémité distale de l'élément de scellage dans la direction de l'axe de l'élément de scellage, les sommets de la pluralité de sommets de la partie cassable sont au nombre de trois.

6. Élément de scellage selon l'une quelconque des revendications 1 à 5, dans lequel le col a une face conique inclinée de telle sorte qu'un diamètre du col diminue progressivement vers le côté de l'emplacement de l'extrémité distale de l'élément de scellage dans la direction de l'axe de l'élément de scellage, et la partie cassable est située sur la face conique.

7. Ensemble poche de sang comprenant :
l'élément de scellage selon l'une quelconque des revendications 1 à 6 ;
une poche de sang qui renferme du sang total ou un composant sanguin ; et
un tube médical en communication avec la poche de sang, dans lequel l'élément de scellage obture le tube médical en contact avec une face interne du tube médical de manière à permettre au tube médical d'être en communication.
